(19) Europäisches Patentamt
European Patent Office
Office européen des brevets

(11) Veröffentlichungsnummer: **0 289 450 A1**

# (12) EUROPÄISCHE PATENTANMELDUNG

(21) Anmeldenummer: **88730101.8**

(22) Anmeldetag: **29.04.88**

(51) Int. Cl.[4]: **C 07 J 31/00**
A 61 K 31/565

(30) Priorität: **30.04.87 DE 3714964**

(43) Veröffentlichungstag der Anmeldung:
**02.11.88 Patentblatt 88/44**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Bohlmann, Rolf, Dr.**
**Melanchtonstrasse 23**
**D-1000 Berlin 21 (DE)**

**Sauer, Gerhard, Dr.**
**Königsbacher Zeile 41 A**
**D-1000 Berlin 28 (DE)**

**Henderson, David, Dr.**
**Jahnstrasse 17**
**D-1000 Berlin 28 (DE)**

**Nishino, Yukishige, Dr.**
**Lanzendorfer Weg 14**
**D-1000 Berlin 22 (DE)**

## (54) 15-Alkyl-19-thio-4-androsten-3,17-dione, Verfahren zu deren Herstellung und diese enthaltende pharmazeutische Präparate.

(57) 15,19-Disubstituierte 4-Androsten-3,17-dione der allgemeinen Formel I

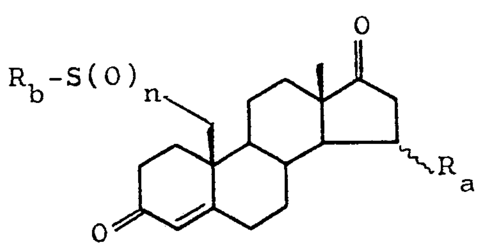

(I),

worin

$R_a$ einen gesättigten oder ungesättigten, gerad- oder verzweigtkettigen Alkylrest mit 1 - 6 Kohlenstoffatomen, wobei $R_a$ in der α- oder β-Position steht,

$R_b$ ein Wasserstoffatom oder einen Alkyl- oder einem Acylrest mit 1 - 6 Kohlenstoffatomen,

n 0, 1 oder 2

bedeuten.

Die neuen Verbindungen der allgemeinen Formel I sind zur Fertilitätskontrolle und zur Behandlung von Krankheiten geeignet, die durch Östrogene hervorgerufen werden.

EP 0 289 450 A1

## Beschreibung

### 15-Alkyl-19-thio-4-androsten-3,17-dione, Verfahren zu deren Herstellung und diese enthaltende pharmazeutische Präparate

Die Erfindung betrifft 15-Alkyl-19-thio-4-androsten-3,17-dione, Verfahren zu deren Herstellung und diese enthaltende pharmazeutische Präparate.

Die erfindungsgemäßen Verbindungen werden durch die allgemeine Formel I beschrieben:

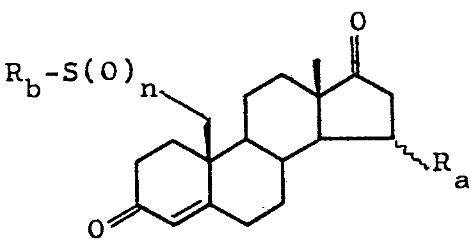

(I),

worin

$R_a$ einen gesättigten oder ungesättigten, gerad- oder verzweigtkettigen Alkylrest mit 1 - 6 Kohlenstoffatomen, wobei $R_a$ in der α- oder β-Position steht,

$R_b$ ein Wasserstoffatom oder einen Alkyl- oder einen Acylrest mit 1 - 6 Kohlenstoffatomen,

n 0, 1 oder 2

bedeuten.

Die für $R_a$ stehenden Alkylgruppen sind gerad- oder verzweigtkettig, gesättigt oder ungesättigt und enthalten 1 - 6 Kohlenstoffatome. Diese Alkylgruppen können am Steroidgerüst α- oder β-ständig sein. Bevorzugt sind die Methyl-, Ethyl-, Propyl-, Butyl-, Hexyl-, 2-Methylpropyl-, 3-Methylbutyl- und Ethenylgruppe.

Die für $R_b$ stehenden Alkyl- und Acylgruppen enthalten 1 - 6 Kohlenstoffatome, beispielsweise die Methyl-, Ethyl-, Propyl-, Hexyl-, Acetyl-, Propionyl- und Hexanoylgruppe.

Die in 19-Stellung stehende Schwefelfunktionalität kann sowohl als Thioether, Sulfoxid oder Sulfon vorliegen.

Die Herstellung der erfindungsgemäßen Verbindungen gemäß Formel I

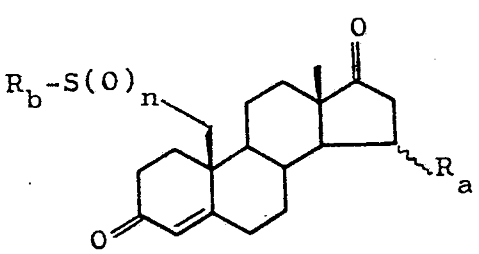

(I),

worin

$R_a$ einen gesättigten oder ungesättigten, gerad- oder verzweigtkettigen Alkylrest mit 1 - 6 Kohlenstoffatomen, wobei $R_a$ in der α- oder β-Position steht,

$R_b$ ein Wasserstoffatom oder einen Alkyl- oder einen Acylrest mit 1 - 6 Kohlenstoffatomen,

n 0, 1 oder 2

bedeuten, erfolgt dadurch, daß man

a) eine Verbindung der allgemeinen Formel II

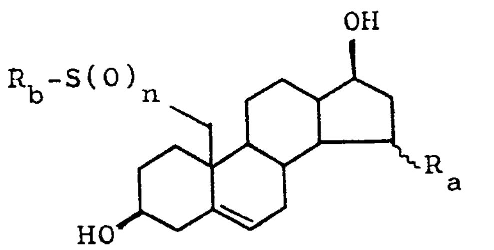

(II),

worin

$R_a$ einen gesättigten oder ungesättigten, gerad- oder verzweigtkettigen Alkylrest mit 1 - 6 Kohlenstoffatomen, wobei $R_a$ in der α- oder β-Position steht,

$R_b$ ein Wasserstoffatom oder einen Alkyl- oder einen Acylrest mit 1 - 6 Kohlenstoffatomen,

n 0, 1 oder 2

bedeuten,

oxidiert oder

b) eine Verbindung der allgemeinen Formel III

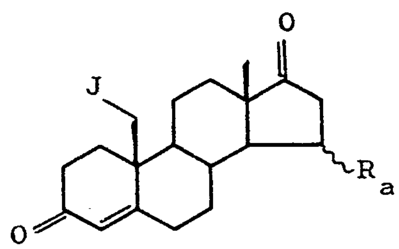

(III),

worin

$R_a$ einen gesättigten oder ungesättigten, gerad- oder verzweigtkettigen Alkylrest mit 1 - 6 Kohlenstoffatomen, wobei $R_a$ in der α- oder β-Position steht,

mit einem Salz des Alkylthiolats oder des Acylthiolats umsetzt und gegebenenfalls oxidiert oder

c) eine Verbindung der allgemeinen Formel IV

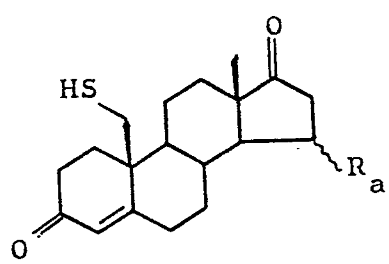

(IV),

worin

$R_a$ einen gesättigten oder ungesättigten, gerad- oder verzweigtkettigen Alkylrest mit 1 - 6 Kohlenstoffatomen, wobei $R_a$ in der α- oder β-Position steht,

bedeuten,

alkyliert oder acyliert und gegebenenfalls oxidiert.

Die Umsetzungen von Verbindungen der allgemeinen Formel II zu Verbindungen der allgemeinen Formel I werden nach Verfahren a) durch Oxydation nach Oppenauer (Org. React. 1951, 6, 207) erreicht.

Die Herstellung der Verbindungen der allgemeinen Formel I aus den Verbindungen der allgemeinen Formel III nach Verfahren b) erfolgt durch Umsetzung eines Alkyl- oder Acylthiolatsalzes in Lösungsmitteln wie Hexamethylphosphorsäuretriamid, Dimethylformamid und Dimethylsulfoxid bei Temperaturen zwischen 0 °C und 150 °C, vorzugsweise bei 60 °C - 100 °C.

Als Salze werden Ammonium-, Alkali- sowie Erdalkalialkylthiolate oder Erdalkaliacylthiolate eingesetzt.

Die 19-Alkylthioether können gegebenenfalls in an sich bekannter Weise in die Sulfoxide oder Sulfone überführt werden. Beispielsweise mittels m-Chlorperbenzoesäure, Wasserstoffperoxid oder Natriumperjodat in inerten Lösungsmitteln, wie Methylenchlorid, Aceton, Acetonitril oder Dimethylformamid.

Die Herstellung der erfindungsgemäßen Verbindungen der allgemeinen Formel I aus den Verbindungen der

allgemeinen Formel IV durch Alkylierung oder Acylierung nach Verfahren c) erfolgt nach literaturbekannten Methoden [Arch. Pharm. (Weinheim) 316, 941, 1983 und Europäische Patentschrift 0 100 566].

Die Verbindungen der allgemeinen Formel II

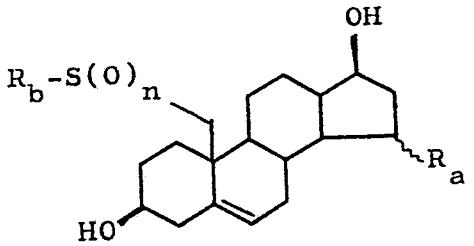

(II)

können ausgehend von 3β-Hydroxy-5,15-androstadien-17-on [J. Chem. Soc. (C) 416, (1968)] auf folgendem Weg hergestellt werden:

Ausgehend von 3β-Hydroxy-5,15-androstadien-17-on gelingt die Einführung der 15β-Cyanogruppe durch Michael-Addition mit Kaliumcyanid; Reduktion des 17-Ketons mit Natriumborhydrid führt zum 3β,17β-Diol. Die 15β-Cyanogruppe läßt sich durch Behandlung mit Kalium-tert.-butylat in Tetrahydrofuran in eine α-Cyanogruppe umwandeln. Beide isomeren Cyanoverbindungen lassen sich mittels Diisobutylaluminiumhydrid sowohl in den 15α-Aldehyd als such in den 15β-Aldehyd überführen. Diese isomeren Aldehyde sind die Ausgangsverbindungen für die 15α- und 15β-substituierten Androstendione. Über die Sequenz Kettenverlängerung durch Wittig-Reaktion, metallkatalysierte Hydrierung der Doppelbindung, Acetylierung des Diols und Reaktion mit N-Bromsuccinimid in Gegenwart von Perchlorsäure erhält man die jeweiligen 3β,17β-Diacetoxy-5α-brom-15(α oder β)-alkyl-6β-androstanole.

Diese Verbindungen lassen sich mit Bleitetraacetat und Jod in die 6β,19-Oxido-Verbindungen überführen. Durch Umsetzung der Oxido-Verbindungen mit Zinkpulver in Eisessig erhält man die 19-Hydroxy-3β,17β-diacetoxy-15-alkyl-5-androstene, welche sich mit Methansulfonsäurechlorid in Pyridin in die 19-Mesylate umwandeln lassen. Durch alkalische Estermethanolyse mit methanolischem Kaliumhydroxyd erhält man die 19-Mesyl-3,17-diole, die sich mit Schwefelnucleophilen, zum Beispiel Natriumthiomethylat, in Verbindungen der allgemeinen Formel II überführen lassen.

Verbindungen der allgemeinen Formel III lassen sich aus den 19-Hydroxy-3β,17β-diacetoxy-15-alkyl-5-androstenen wie folgt herstellen:

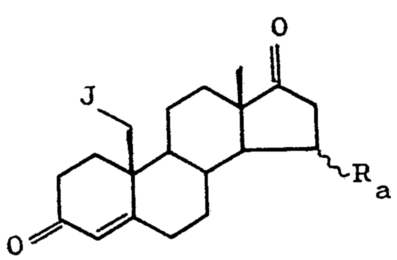

(III).

Die 19-Hydroxygruppe kann durch Behandeln mit Dihydropyran unter sauren Bedingungen als THP-ether geschützt werden. Durch alkalische Esterspaltung mit methanolischer Kaliumhydroxidlösung kann man das entsprechende 3β,17β-Diol herstellen, welches nach Oppenauer durch Behandlung mit einem Keton in Gegenwart von Aluminiumtriisopropylat zum 3,17-Dion überführt werden kann. Das 3,17-Dion läßt sich mit methanolischer Salzsäure in den 19-Alkohol überführen, der mit Trifluormethansulfonsäureanhydrid in das 19-Triflat übergeht. Durch Umsetzung des Triflats mit Natriumjodid in Cyclopentanon [NL-B- 65 05 443 (8.10.1965)] gelingt die Bereittung der 19-Jodverbindung der allgemeinen Formel III.

Die Verbindungen der allgemeinen Formel IV lassen sich wie folgt herstellen:

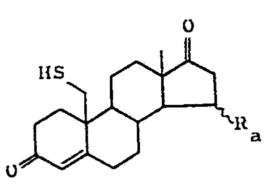

(IV)

Durch Behandeln mit Kaliumthioacetat werden die Verbindungen der allgemeinen Formel III in die 19-Acetylthioverbindungen überführt, die durch alkalische Methanolyse mit methanolischer Natriummethanolatlösung in die Verbindungen der allgemeinen Formel IV umgesetzt werden.

Die Verbindungen der allgemeinen Formel I sind Inhibitoren der Östrogenbiosynthese (Aromatasehemmer). Sie sind damit zur Behandlung von Krankheiten geeignet, die durch Östrogene bedingt oder von Östrogenen abhängig sind. So sind sie geeignet zur Behandlung von östrogeninduzierten oder -stimulierten Tumoren, wie zum Beispiel Mammakarzinom (The Lancet, 1984, 1237-1239) oder Prostatahyperplasie.

Die erfindungsgemäßen Verbindungen sind auch wertvoll zur Beeinflussung der Fertilität. So kann eine männliche Infertilität, die aus erhöhten Östrogenspiegeln resultiert, mit den neuen Wirkstoffen behoben werden.

Ferner können die Verbindungen bei Frauen im fortpflanzungsfähigen Alter als Antifertilitätsmittel verwendet werden, um Ovulation oder Implantation durch Östrogenentzug zu hemmen.

Wahrscheinlich sind Aromatasehemmer auch zur Behandlung des drohenden Herzinfarkts geeignet, da erhöhte Östrogenspiegel beim Mann einem Herzinfarkt vorangehen können (US-Patent 4,289,762).

Bekannte, eine die Aromatase hemmende Wirkung aufweisende Steroidsubstanzen sind beispielsweise 19-Methylthio-4-androsten-3,17-dione (EP-Patent 0 100 566).

Im Vergleich zu den bekannten Aromatasehemmern wird die Serum-Östradiolkonzentration mit den erfindungsgemäßen Verbindungen bei oraler Applikation stärker gesenkt.

So konnte zum Beispiel im sogenannten PMSG (Pregnant Mare's Serum Gonadotropin)-Test [J. Steroid. Biochem. 7, (1976) 787] gezeigt werden, daß im Vergleich zu dem bekannten Aromatasehemmer 19-Methylthio-4-androsten-3,17-dion (A) die erfindungsgemäßen Verbindungen, wie zum Beispiel 15α-Ethyl-19-methylthio-4-androsten-3,17-dion (B), bei oraler Applikation eine stärkere Wirkung besitzen.

Infantile weibliche Ratten reagieren auf PMSG-Behandlung mit einheitlich erhöhter Steroidsynthese. Die Aromataseaktivität kann durch die Beeinflussung der PMSG-stimulierten Östrogenbildung gemessen werden.

Im PMSG-Test werden 21 Tage alte weibliche Ratten alle 2 Tage insgesamt 7 mal mit je 100 I.U. PMSG subkutan vorbehandelt. 1 Stunde vor und 8 Stunden nach der letzten PMSG-Applikation ($d_{12}$) erhalten die Tiere die Testsubstanz durch orale Applikation.

Die Kontrolltiere erhalten nur das Vehikel. 24 Stunden nach der letzten PMSG-Applikation werden die Tiere getötet. Im Serum wird Östradiol radioimmunologisch geprüft. Für jede Gruppe von 10 Tieren wird der Mittelwert der Östradiolkonzentration in nMol/l mit der Standardabweichung errechnet. Die Signifikanzen der Unterschiede zur Kontrollgruppe werden durch die Varianzanalyse geprüft.

Zur Ermittlung der relativen Wirkungsstärke der zu testenden Substanz im Vergleich zu der Vergleichssubstanz wird die Regressions- und Kovarianzanalyse durchgeführt. Ferner wird die prozentuale Hemmung gegen die PMSG-Kontrolle berechnet.

Am Beispiel der erfindungsgemäßen Verbindungen 15α-Ethyl-19-methylthio-4-androsten-3,17-dion (B) wird gezeigt, daß die erfindungsgemäßen Verbindungen die Östradiolkonzentration im Serum weit stärker senken als das 19-Methylthio-4-androsten-3,17-dion (A). Während eine Hemmung der Östradiolkonzentration bei der erfindungsgemäßen Verbindung deutlich mit 35 % gefunden wurde, hemmt die Vergleichssubstanz mit 11 % nur schwach. Für die Verbindung B wird eine relative Wirkstärke von 3,2 gegenüber Verbindung A gefunden.

T a b e l l e

Beeinflussung der Östradiol-Konzentration im peripheren Serum bei der PMSG-vorbehandelten Ratte

| | Dosis mg/Tier 2 x p.o. | n | Östradiol-Konzentration in | Hemmung % | relative Wirkstärke |
|---|---|---|---|---|---|
| PMSG-Kontrolle | - | 10 | 6,20 ± 1,13 | - | - |
| A | 10 | 10 | 5,50 ± 0,43 | 11 | 1,0 |
| B | 10 | 10 | 4,05 ± 0,36 | 35 | 3,2 |

n = Tierzahl pro Gruppe

Die zu verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,001 - 100 mg/kg Körpergewicht, vorzugsweise 0,01 - 20 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten. Die einzelnen Dosierungseinheiten für die orale Applikation können beispielsweise 10 bis 100 mg des Wirkstoffs (Aromataseinhibitors) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Als Öle werden zum Beispiel Olivenöl, Erdnußöl, Baumwollsamenöl, Sojabohnenöl, Rizinusöl und Sesamöl verwendet.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone, wie zum Beispiel Silikonkautschuk. Die Wirkstoffe können außerdem zur perkutanen Applikation zum Beispiel in ein Pflaster eingearbeitet werden.

Die Erfindung betrifft somit auch pharmazeutische Präparate und die Verwendung der neuen Verbindungen der allgemeinen Formel I zur Herstellung dieser Präparate zur Behandlung von östrogen-bedingten Krankheiten.

**Herstellung der Ausgangsverbindungen**

1.) 15α-Ethyl-19-methylthio-5-androsten-3β,17β-diol

a) 50,3 g 3β-Hydroxy-5,15-androstadien-17-on [R.W. Kelly and P.J. Sykes, J. Chem. Soc. (C), 416 (1968)] werden in 1,06 l Tetrahydrofuran mit 60,8 g Kaliumcyanid in 350 ml Wasser versetzt und 15 Minuten am Rückfluß gekocht. Dann wird mit Wasser verdünnt und zweimal mit Essigester extrahiert. Man erhält 53 g 15β-Cyano-3β-hydroxy-5-androsten-17-on als Rohprodukt.

b) 22,8 g 15β-Cyano-3β-hydroxy-5-androsten-17-on werden in 600 ml Methanol und 300 ml Tetrahydrofuran mit 11,5 g Natriumborhydrid und 46 ml Wasser portionsweise versetzt und 15 Minuten bei 20 °C gerührt. Dann wird in 10 l Eiswasser gegossen, der Niederschlag abgesaugt, mit Wasser neutral

gewaschen und im Trockenschrank bei 70 °C über Nacht getrocknet. Das Rohprodukt wird aus Aceton/Toluol (1:1) kristallisiert. Man erhält 20,6 g 15β-Cyano-5-androsten-3β,17β-diol vom Schmelzpunkt 251 - 255 °C.

c) 18,6 g 15β-Cyano-5-androsten-3β,17β-diol werden in 700 ml Tetrahydrofuran unter Argon mit 36 g Kalium-tert.-butylat in 300 ml Tetrahydrofuran 15 Minuten am Rückfluß gekocht. Beim Abkühlen kristallisiert das Reaktionsprodukt aus. Durch Absaugen der Kristalle werden 16,4 g 15α-Cyano-5-androsten-3β,17β-diol erhalten.

d) 5 g 15α-Cyano-5-androsten-3β,17β-diol werden in 200 ml Tetrahydrofuran bei -15 °C mit 150 ml Diisobutylalumiumhydridlösung (1,0 M in Tetrahydrofuran) tropfenweise versetzt und 2 Stunden bei 0 °C nachgerührt. Dann werden 300 ml 10 % wäßrige Weinsäure portionsweise zugegeben, in 400 ml Wasser und 200 g Eis eingefällt, abgesaugt, mit Wasser gewaschen und getrocknet. Nach der Kristallisation aus Aceton erhält man 4,0 g 3β,17β-Dihydroxy-5-androsten-15α-carbaldehyd vom Schmelzpunkt 171 - 173 °C (Zersetzung).

e) 19,7 g 3β,17β-Dihydroxy-5-androsten-15α-carbaldehyd werden bei 20 °C zu einer Mischung von 56 g Triphenylmethylphosphoniumbromid in 490 ml Dimethylsulfoxid und 17,6 g Kalium-tert.-butylat gegeben und 2 Stunden bei 20 °C gerührt. Dann wird mit verdünnter Schwefelsäure gefällt und zweimal mit Essigester extrahiert. Die vereinigte organische Phase wird mit Waser und Natriumchloridlösung gewaschen, über Natriumsulfat getrocknet und im Vakuum eingeengt. Das erhaltene Produkt wird in 500 ml 0,2 n methanolischer Kaliumhydroxid-Lösung 3,5 Stunden bei 20 °C gerührt. Danach wird mit Essigsäure neutralisiert, mit Eiswasser gefällt, mit Wasser gewaschen und im Vakuum bei 70 °C eingeengt. Man erhält 32 g rohes 15α-Vinyl-5-androsten-3β,17β-diol, das mit Triphenylphosphin verunreinigt ist.

f) 32 g rohes 15α-Vinyl-5-androsten-3β,17β-diol werden in 450 ml Methanol mit 3,2 g Palladium (10 %) auf Aktivkohle 1,5 Stunden unter Wasserstoff geschüttelt. Der Katalysator wird über Kieselgur abfiltriert, mit Dichlormethan nachgewaschen und die organische Phase im Vakuum eingeengt. Man erhält 32 g rohes 15α-Ethyl-5-androsten-3β,17β-diol.

g) 3,9 g 15α-Ethyl-5-androsten-3β,17β-diol in 50 ml Pyridin werden mit 25 ml Essigsäureanhydrid 17 Stunden bei 20 °C gerührt. Dann wird mit salzsaurem Sole/Eiswasser gefällt, abfiltriert, mit Wasser gewaschen, in Dichlormethan aufgenommen, neutral gewaschen, über Natriumsulfat getrocknet und eingeengt. Nach der chromatographischen Reinigung an Kieselgel mit Hexan/Essigester erhält 4,1 g 3,17-Diacetoxy-15α-ethyl-5-androsten.

h) 4,1 g 3β-17β-Diacetoxy-15α-ethyl-5-androsten in 62 ml Diethylether werden bei 0 °C mit 3,1 g N-Bromsuccinimid und mit einer Lösung aus 18 ml 70 %iger Perchlorsäure und 44 ml Wasser versetzt und 0,5 Stunden gerührt. Dann wird mit Natriumsulfatlösung versetzt, mit Essigester extrahiert, neutral gewaschen, über Natriumsulfat getrocknet und im Vakuum eingeengt. Man erhält 5,0 g rohes 3β,17β-Diacetoxy-5α-brom-15α-ethyl-6β-androstanol.

i) 5,0 g 3β,17β-Diacetoxy-5α-brom-15α-ethyl-6β-androstanol in 175 ml Cyclohexan werden mit 2,5 g Bleitetracetat und 1,6 g Jod 2,5 Stunden am Rückfluß gesiedet. Dann wird der Feststoff abfiltriert, mit Essigester gewaschen, das Filtrat mit Natriumthiosulfat, Wasser und Natriumchloridlösung gewaschen, über Natriumsulfat getrocknet und im Vakuum eingeengt. Man erhält 4,8 g rohes 3β,17β-Diacetoxy-5α-brom-15α-ethyl-6β,19-oxidoandrostan.

j) 4,8 g rohes 3β,17β-Diacetoxy-5α-brom-15α-ethyl-6β,19-oxidoandrostan in 80 ml Eisessig werden mit 9,6 g Zink 3 Stunden bei 20 °C gerührt. Dann wird das Zink abgenutscht, mit Essigester gewaschen, mit Natriumhydrogencarbonat- und Natriumchloridlösung neutral gewaschen, über Natriumsulfat getrocknet und eingeengt. Nach der chromatographischen Reinigung an Kieselgel mit Hexan/Essigester erhält man 950 mg reines 3β,17β-Diacetoxy-15α-ethyl-19-hydroxy-5-androsten als Schaum.

k) 0,9 g 3β,17β-Diacetoxy-15α-ethyl-19-hydroxy-5-androsten in 10 ml Pyridin werden bei 0 °C mit 1,5 ml Methansulfonsäurechlorid 3,5 Stunden gerührt. Die Aufarbeitung erfolgt analog g). Man erhält 920 mg rohes 3β,17β-Diacetoxy-15α-ethyl-19-mesyloxy-5-androsten.

l) 920 mg 3β,17β-Diacetoxy-15α-ethyl-19-mesyloxy-5-androsten werden 3 Stunden bei 20 °C in 12 ml einer 0,2 n methanolischen Kalilauge gerührt. Dann wird mit Wasser verdünnt, mit Essigester extrahiert, über Natriumsulfat getrocknet und im Vakuum zur Trockne eingeengt. Man erhält 788 mg rohes 15α-Ethyl-19-mesyloxy-5-androsten-3β,17β-diol.

m) 0,78 g rohes 15α-Ethyl-19-mesyloxy-5-androsten-3β-17β-diol in 15 ml Dimethylformamid werden mit 548 mg Natriummethylthiolat 1,5 Stunden bei 70 °C gerührt. Dann wird mit Sole/Eiswasser gefällt, abfiltriert, mit Wasser gewaschen, in Essigester aufgenommen, neutral gewaschen, über Natriumsulfat getrocknet und im Vakuum eingeengt. Man erhält nach der chromatographischen Reinigung 430 mg reines 15α-Ethyl-19-methylthio-5-androsten-3β,17β-diol.

2.) 15β-Ethyl-19-methylthio-5-androsten-3β,17β-diol

50 g rohes 15β-Cyano-3β-hydroxy-5-androsten-17-on werden in 950 ml Tetrahydrofuran bei -20 °C mit 950 ml Diisobutylaluminiumhydrid-Lösung (1,2 M in Toluol) 2,5 Stunden gerührt. Dann wird mit verdünnter Schwefelsäure versetzt, über Kieselgur abgesaugt, die organische Phase mit Natriumhydrogencarbonatlösung neutral gewaschen, über Natriumsulfat getrocknet und im Vakuum eingeengt. Es werden 50,1 g roher 3β,17β-Dihydroxy-5-androsten-15β-carbaldehyd erhalten.

Die weitere Herstellung zum 15β-Ethyl-19-methylthio-5-androsten-3β,17β-diol erfolgt analog der Herstellung der Ausgangsverbindungen 1 e) - m).

**Beispiel 1**

430 mg 15α-Ethyl-19-methylthio-5-androsten-3β,17β-diol in 31 ml Toluol werden mit 11,5 ml Cyclohexanon und 430 mg Aluminiumtriisopropylat 4 Stunden am Wasserabscheider erhitzt. Dann wird im Vakuum vom Lösungsmittel befreit und an Kieselgel mit Hexan/Essigester chromatographiert. Mab erhält 300 mg reines 15α-Ethyl-19-methylthio-4-androsten-3,17-dion vom Schmelzpunkt 151 - 152 °C.
UV: $\varepsilon_{240}$ 14270.

**Beispiel 2**

15β-Ethyl-19-methylthio-4-androsten-3,17-dion

Analog Beispiel 1 wird 15β-Ethyl-19-methylthio-4-androsten-3,17-dion mit Schmelzpunkt 145 - 146 °C erhalten.

**Patentansprüche**

1.) 15,19-Disubstituierte 4-Androsten-3,17-dione der allgemeinen Formel I

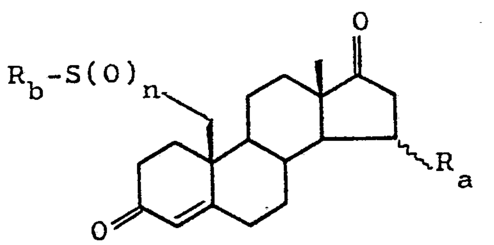

(I),

worin
$R_a$ einen gesättigten oder ungesättigten, gerad- oder verzweigtkettigen Alkylrest mit 1 - 6 Kohlenstoffatomen, wobei $R_a$ in der α- oder β-Position steht,
$R_b$ ein Wasserstoffatom oder einen Alkyl- oder einen Acylrest mit 1 - 6 Kohlenstoffatomen,
n 0, 1 oder 2
bedeuten.

2.) 15α-Ethyl-19-methylthio-4-androsten-3,17-dion.

3.) 15β-Ethyl-19-methylthio-4-androsten-3,17-dion.

4.) Pharmazeutische Präparate, gekennzeichnet durch den Gehalt an einer Verbindung gemäß Anspruch 1 - 3.

5.) Verwendung der Verbindungen gemäß Anspruch 1 - 3 zur Herstellung von Präparaten zur Behandlung von östrogen-bedingten Krankheiten.

6.) Verfahren zur Herstellung von 15,19-disubstituierten 4-Androsten-3,17-dionen der allgemeinen Formel I

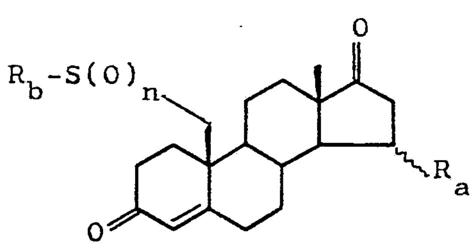

(I),

worin
$R_a$ einen gesättigten oder ungesättigten, gerad- oder verzweigtkettigen Alkylrest mit 1 - 6 Kohlenstoffatomen, wobei $R_a$ in der α- oder β-Position steht,
$R_b$ ein Wasserstoffatom oder einen Alkyl- oder einen Acylrest mit 1 - 6 Kohlenstoffatomen,
n 0, 1 oder 2

bedeuten, dadurch gekennzeichnet, daß man

a) eine Verbindung der allgemeinen Formel II

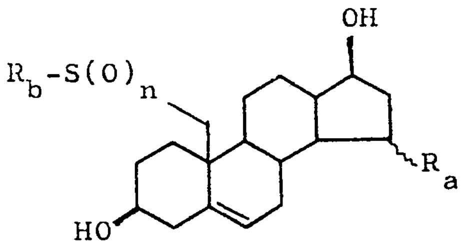

(II),

worin

$R_a$ einen gesättigten oder ungesättigten, gerad- oder verzweigtkettigen Alkylrest mit 1 - 6 Kohlenstoffatomen, wobei $R_a$ in der α- oder β-Position steht.

$R_b$ ein Wasserstoffatom oder einen Alkyl- oder einen Acylrest mit 1 - 6 Kohlenstoffatomen,

n 0, 1 oder 2

bedeuten,

oxidiert oder

b) eine Verbindung der allgemeinen Formel III

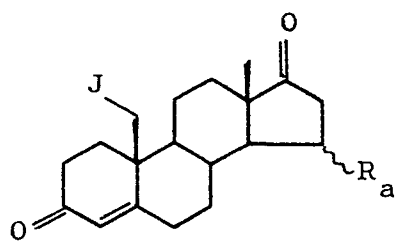

(III),

worin

$R_a$ einen gesättigten oder ungesättigten, gerad- oder verzweigtkettigen Alkylrest mit 1 - 6 Kohlenstoffatomen, wobei $R_a$ in der α- oder β-Position steht,

mit einem Salz des Alkylthiolats oder des Acylthiolats umsetzt und gegebenenfalls oxidiert oder

c) eine Verbindung der allgemeinen Formel IV

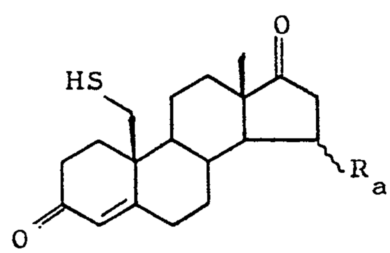

(IV),

worin

$R_a$ einen gesättigten oder ungesättigten, gerad- oder verzweigtkettigen Alkylrest mit 1 - 6 Kohlenstoffatomen, wobei $R_a$ in der α- oder β-Position steht,

bedeuten,

alkyliert oder acyliert und gegebenenfalls oxidiert.

Europäisches Patentamt

# EUROPÄISCHER RECHERCHENBERICHT

Nummer der Anmeldung

EP 88 73 0101

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 100 566 (AKZO)<br>* Ansprüche *<br>--- | 1,4-6 | C 07 J 31/00<br>A 61 K 31/565 |
| A | EP-A-0 149 499 (AKZO)<br>* Ansprüche *<br>----- | 1,4-6 | |
| | | | **RECHERCHIERTE SACHGEBIETE (Int. Cl.4)** |
| | | | C 07 J 31/00 |

Der vorliegende Recherchenbericht wurde für alle Patentansprüche erstellt

| Recherchenort | Abschlußdatum der Recherche | Prüfer |
|---|---|---|
| DEN HAAG | 28-07-1988 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................
& : Mitglied der gleichen Patentfamilie, übereinstimmendes Dokument

EPO FORM 1503 03.82 (P0403)